# EUROPEAN PATENT SPECIFICATION

(11) **EP 2 641 889 B1**
(45) Date of publication and mention of the grant of the patent: **20.12.2017**
(21) Application number: 11841370.7
(22) Date of filing: 18.11.2011
(51) Int. Cl.: C07C 29/154, B01J 23/80, C07C 31/04, C07B 61/00

(54) **METHANOL PRODUCTION PROCESS**
VERFAHREN ZUR HERSTELLUNG VON METHANOL
PROCÉDÉ DE PRODUCTION DE MÉTHANOL

(30) Priority: 19.11.2010 JP 2010258866
(43) Date of publication of application: 25.09.2013
(73) Proprietor: Mitsui Chemicals, Inc., Tokyo 105-7117 (JP)
(72) Inventor: MATSUSHITA, Tatsumi, Takaishi-shi Osaka 592-8501 (JP); HAGANUMA, Tsukasa, Takaishi-shi Osaka 592-8501 (JP); FUJITA, Daisuke, Takaishi-shi Osaka 592-8501 (JP)
(74) Representative: J A Kemp
(86) International application number: PCT/JP2011/076641
(87) International publication number: WO 2012/067222

(56) References cited:
- WO-A1-2005/021474
- DE-A1- 4 227 484
- JP-A- 10 309 466
- JP-A- 11 188 262
- JP-A- 57 106 629
- JP-A- 59 205 336
- JP-A- 2004 315 473
- JP-A- 2007 502 835
- JP-A- 2009 538 907

## Description

### TECHNICAL FIELD

The present invention relates to a process for producing methanol from carbon dioxide and hydrogen as materials.

### BACKGROUND ART

Methanol is used as a material for products such as dimethyl ether, MTBE (methyl tertiary butyl ether) and petrochemical intermediates, and also as a fuel.

In a conventional methanol production process, fossil fuels such as hydrocarbons and cokes as materials are steam reformed into a syngas (containing CO, H₂ and a small amount of CO₂), and the syngas is reacted as a main material to synthesize methanol. Such a process relies on the use of fossil materials as main ingredients. Thus, the process consumes earth resources and increases the amount of CO₂ in the atmosphere, thereby contributing to global warming.

A known alternative technique is to synthesize methanol from CO₂ and hydrogen as materials. According to this process, carbon dioxide that is a factor of global warming is converted into the organic compound. Thus, this technique is highly desirable not only in view of the fact that it contributes to various chemical production activities but also from the viewpoint of the reduction of carbon dioxide blamed for global warming, namely, from the viewpoint of the prevention of global warming.

Due to the thermodynamic equilibrium of the reaction as well as because the reaction is inhibited by water that is by-produced together with methanol, synthesizing methanol from a material gas with a high carbon dioxide content requires a catalyst exhibiting a higher activity and a higher durability than in the methanol synthesis from a syngas.

With such needs, copper-containing multicomponent catalysts such as copper/zinc oxide/aluminum oxide/zirconium oxide and copper/zinc oxide/aluminum oxide/zirconium oxide/gallium oxide have been developed (see, for example, JP-A-H07-39755 (Patent Literature 1), JP-A-H06-312138 (Patent Literature 2), and Applied Catalysis A: General, 138, 311-318 (1996) (Non-Patent Literature 1)). Further, JP-A-H10-309466 (Patent Literature 3) discloses a highly active catalyst which contains 0.3 to 0. 9 wt% of silica derived from a colloidal silica or a water-dissolved silica and which is prepared by calcination at 480 to 690°C.

Because these catalysts are solid catalysts, the methanol synthesis reaction is generally carried out with a fixed-bed reactor. In general, the reactor may be a multitubular reactor. In general, the reaction mode may be batchwise. It will also be preferable to adopt a continuous reaction mode in which part or all of unreacted materials (including carbon dioxide and hydrogen) are recovered from the reaction mixture containing methanol formed, and the unreacted material components are circulated back to the material introduction step. In order to separate the reaction mixture into methanol and the unreacted gas, techniques such as distillation and separation with a gas-liquid separator are known.

### Citation List

### Patent Literatures

Patent Literature 1: JP-A-1995-39755
Patent Literature 2: JP-A-1994-312138
Patent Literature 3: JP-A-1998-309466

### Non-Patent Literature

Non-Patent Literature 1: Applied Catalysis A: General, 138, 311-318 (1996)

### SUMMARY OF INVENTION

### Technical Problem

According to studies carried out by the present inventors, methanol production from carbon dioxide and hydrogen has suffered a decrease in reaction efficiency with time when unreacted materials are recovered and reused as materials.

It has been found that the above phenomenon is ascribed partly to the degradation of the catalyst with time but is attributed largely to the fact that the proportion of components which are present in small amounts in the reaction materials and do not react with the catalyst of the present application (hereinafter, sometimes referred to as inert components) is increased with time with the result that carbon dioxide and hydrogen to be reacted with each other under catalysis of the catalyst come to represent a smaller proportion relative to the materials supplied to the reactor.

It has been further found that the inert components include compounds containing carbon and hydrogen, such as methane. Furthermore, the present inventors have found that hydrogen and carbon dioxide used as materials contain such inert components in small amounts. That is, the progress of the methanol production reaction is accompanied by an increasing proportion of the inert components and a consequent decreasing concentration of the materials, thus leading to a decrease in reaction efficiency.

A possible approach to remedy this decrease in reaction efficiency is to clear the reactor system of part or all of the unreacted materials that have come to have a larger proportion of the inert components. However, such an approach causes a risk that the inert components such as methane are released into the atmosphere. Methane is known to have a greenhouse effect. Thus, this approach can adversely affect the global environment.

Accordingly, it is important that a methanol production process be provided which can efficiently produce methanol from carbon dioxide and hydrogen while suppressing loads to the global environment. The present invention has been made in order to address this problem.

### Solution to Problem

In view of the above problem, the present inventors have carried out studies. As a result, they have found that methanol can be produced with good efficiency and the above problem can be solved by providing a step in which part or all of unreacted materials containing the inert components are combusted to produce a combustion product and energy such as thermal energy, and a step in which the combustion product from the above step which contains carbon oxides such as carbon monoxide and carbon dioxide is circulated again as a material. The present invention has been completed based on this finding.

A process for producing methanol according to the present invention comprises a step (a) of reacting hydrogen and carbon dioxide with each other in a reactor in the presence of a copper-containing catalyst to produce a reaction mixture containing methanol;
a step (b) of circulating part or all of the reaction mixture to the step (a); and then
a step (c1) of combusting part of the reaction mixture to produce a combustion product and energy, and a step (c2) of circulating part or all of the combustion product to the step (a), the step (c1) and the step (c2) being performed when the reaction mixture contains a compound (p) containing carbon and hydrogen except methanol at not less than 0.1 mol% (wherein all components of the reaction mixture excluding methanol and water represent 100 mol%); and
a step (d) of separating a component including methanol from the reaction mixture.

The compound (p) is preferably inert to the copper-containing catalyst.

It is preferable that at least part of the compounds (p) be a hydrocarbon, and more preferably methane. It is particularly preferable that the compound (p) be methane.

Preferably, the reaction mixture contains the compound (p) at 0.1 to 50 mol%.

The copper-containing catalyst is preferably a catalyst containing copper, zinc, aluminum and silicon.

The energy produced in the step (c1) is preferably recovered in the form of energy selected from thermal energy, electric energy and kinetic energy, and is more preferably recovered as thermal energy. Advantageous Effects of Invention

According to the methanol production process of the present invention, an increase in the proportion of inert components, in detail, compounds containing carbon and hydrogen except methanol, with time during the methanol production steps is addressed by combusting the compounds when the concentration of the compounds reaches a specific concentration, thereby reducing the amount of the inert components or eliminating the inert components. Thus, the methanol production process of the invention can suppress an increase with time of the concentration of the inert components present in the reaction system, thereby allowing for efficient production of methanol.

Further, according to the methanol production process of the invention, the energy produced by combusting the compounds can be recovered. Furthermore, according to the methanol production process of the invention, carbon oxides such as carbon monoxide and carbon dioxide resulting from the combustion can be reused as materials for the production of methanol.

The methanol production process of the invention is of great significance in industry also from the viewpoint of global environment protection because the process allows for, in addition to efficient methanol production, the reuse of carbon oxides such as carbon monoxide and carbon dioxide resulting from combustion.

### Brief Description of Drawings

[FIG. 1] FIG. 1 is a flowchart diagram illustrating a system used in EXAMPLES.
[FIG. 2] FIG. 2 is a flowchart diagram illustrating a system used in COMPARATIVE EXAMPLES.

### DESCRIPTION OF EMBODIMENTS

The present invention will be described in detail hereinbelow.

A process for producing methanol according to the present invention includes a step (a) of reacting hydrogen and carbon dioxide with each other in a reactor in the presence of a copper-containing catalyst to produce a reaction mixture containing methanol; a step (b) of circulating part or all of the reaction mixture to the step (a) ; a step (c1) of combusting part of the reaction mixture to produce a combustion product and energy, and a step (c2) of circulating part or all of the combustion product to the step (a), the step (c1) and the step (c2) being performed when the reaction mixture contains a compound (p) containing carbon and hydrogen except methanol at not less than 0.1 mol% (wherein all the components of the reaction mixture excluding methanol and water represent 100 mol%); and a step (d) of separating a component including methanol from the reaction mixture.

The reactor is usually a fixed-bed reactor in which the catalyst is packed in a fixed bed. In such a case, the reactor includes a catalyst layer.

The hydrogen and the carbon dioxide used in the invention may be obtained by known methods without limitation. For example, the hydrogen may be any known hydrogen such as hydrogen generated (by-produced) by a steam reforming reaction, hydrogen generated by electrolysis, or hydrogen obtained by photolysis of water. For example, the carbon dioxide may be one generated by any of various chemical reactions or by the combustion of a fuel in petrochemical plants. It tends to be substantially impossible for these hydrogen and carbon dioxide to be free of compounds (p) containing carbon and hydrogen as will be described later.

In the step (a) of the inventive methanol production process, carbon dioxide and hydrogen are reacted with each other to synthesize methanol and water. The reaction mixture obtained by the reaction contains methanol and water, and usually further contains unreacted materials (for example, hydrogen, carbon dioxide) and byproducts (for example, carbon monoxide). Further, the reaction mixture often contains inert components, in detail, compounds (p) containing carbon and hydrogen except methanol, for example hydrocarbons such as methane and ethane.

In the methanol production process of the invention, generally, a component including methanol is separated from the obtained reaction mixture. In detail, the unreacted materials, the byproducts and the compounds (p) are separated from the reaction mixture, thereby obtaining a mixture of methanol and water as the component including methanol.

Further, methanol is obtained usually by dehydrating the mixture of methanol and water. The dehydration method is not particularly limited and may be any of known methods such as distillation.

In the step (b) of the inventive methanol production process, part or all of the reaction mixture is circulated to the step (a). The part of the reaction mixture that is circulated may be circulated in such a manner that the reaction mixture obtained in the step (a) is directly circulated. Alternatively, the residue after the separation of the component including methanol from the reaction mixture, namely, the residue composed of the unreacted materials, the byproducts and the compounds (p) may be circulated. Because the reaction between hydrogen and carbon dioxide in the invention is an equilibrium reaction, the latter case is preferable. That is, it is preferable that the residue after the separation of methanol be circulated. By performing the step (b) in this manner, the reaction efficiency may be improved.

In the present invention, hydrogen and carbon dioxide are reacted in such a manner that hydrogen and carbon dioxide are brought into contact with a catalyst described later to induce the reaction. When hydrogen and carbon dioxide are brought into contact with the catalyst, the molar ratio of hydrogen to carbon dioxide (hydrogen/carbon dioxide) is preferably not less than 2.5, and more preferably not less than 3.0. In the case where a fixed-bed reactor is used in which the catalyst is packed in a fixed bed, the hydrogen to carbon dioxide ratio is more preferably not less than 3.5, and particularly preferably not less than 4.0.

The reaction between hydrogen and carbon dioxide to form methanol takes place in accordance with the following reaction formula.

3H₂ + CO₂ → CH₃OH + H₂O

If the hydrogen to carbon dioxide molar ratio is below the above range, methanol productivity is lowered as apparent from the above reaction formula as well as from the fact that the loss of pressure through the catalyst layer is so increased in the case of a fixed-bed reactor that the equilibrium conversion is decreased. The upper limit of the hydrogen to carbon dioxide molar ratio at the contact with the catalyst layer is not particularly limited. However, an excessively high hydrogen to carbon dioxide molar ratio can result in a decrease in methanol productivity. Thus, the upper limit of the hydrogen to carbon dioxide molar ratio is preferably 20.0, more preferably 10.0, still more preferably 6.0, particularly preferably 5.5, and further preferably 5.0.

In the methanol production process of the invention, water is produced together with methanol. In general, water is adsorbed onto a catalyst to inhibit the methanol forming reaction, and thus can be a factor that decreases the efficiency in methanol production. When the hydrogen to carbon dioxide ratio is high, for example 3.5 or more, the supply of hydrogen relative to that of carbon dioxide is large and consequently the formed water is diluted to a lower water concentration than a conventional level. Because a decrease in water concentration leads to a smaller amount of water adsorbed onto a catalyst, such a molar ratio suppresses a decrease in methanol production efficiency caused by water. This is probably the reason why the inventive methanol production process performed under the above conditions achieves a high efficiency in methanol production.

In the methanol production process of the invention, hydrogen and carbon dioxide are reacted with each other in the presence of a copper-containing catalyst. The copper-containing catalyst may be any of known catalysts without limitation as long as the catalyst contains copper and can catalyze the reaction between hydrogen and carbon dioxide into methanol.

Preferred examples of the copper-containing catalysts for use in the invention include catalysts described in Patent Literature 3 which contain copper, zinc, aluminum and silicon as essential components and have zirconium, palladium and gallium as optional components. The copper-containing catalyst used in the invention is preferably a catalyst containing copper, zinc, aluminum and silicon, and is more preferably a catalyst containing copper, zinc, aluminum and silicon as well as at least one metal selected from zirconium, palladium and gallium. These catalysts containing copper and other components may be suitably used in the methanol production process of the invention for reasons such as a small decrease in activity by water which is by-produced during the production of methanol from carbon dioxide.

The copper-containing catalyst preferably has a particle diameter of 0.5 to 20 mm, more preferably 1 to 20 mm, still more preferably 2 to 20 mm, even more preferably 3 to 20 mm, further preferably 3 to 15 mm, and particularly preferably 3 to 10 mm. This particle diameter of the copper-containing catalyst ensures not only that the catalyst is handled easily but also that the occurrence of a pressure loss is suppressed when methanol is produced by the inventive method using a catalyst layer in which the catalyst is packed in a fixed bed. The catalyst having the above particle diameter may be produced by any of known methods without limitation. A tableting method is suitably used.

The reactor used in the step (a) of the inventive production process may be any of known reactors without limitation. For example, it is preferable that the copper-containing catalyst be packed in a fixed-bed reactor. Alternatively, a radial flow reactor may be suitably used. Because the inventive process can decrease a pressure loss, good productivity is obtained even with a relatively large radial flow reactor, thus providing a possibility of efficient methanol production.

In a preferred embodiment for carrying out the step (a) of the inventive production process, hydrogen and carbon dioxide are reacted with each other in a reactor which has a catalyst layer formed of the copper-containing catalyst. In the reaction, hydrogen and carbon dioxide are supplied from upstream of the catalyst layer in the reactor, and a reaction mixture containing methanol is obtained from downstream of the catalyst layer. The thickness of the catalyst layer in the reactor is preferably not less than 1 m. The thickness of the catalyst layer is preferably not less than 2 m, more preferably not less than 3 m, and still more preferably not less than 4 m. Specifying the upper limit of the thickness does not have any positive significance. However, the catalyst layer with an excessively large thickness tends to have a large adverse effect in terms of pressure loss. Thus, the upper limit is preferably 20 m, and more preferably 15 m. The thickness of the catalyst layer is not necessarily determined by the positional relationship between the upstream side and the downstream side, but means a substantial length determined in consideration of the shape of the catalyst layer. It is not necessary that the thickness of the catalyst layer be identical at any given positions. It is preferable that substantially the thinnest portion satisfy the above thickness. When the catalyst layer has a thinner portion, it is probable that material gases such as carbon dioxide and hydrogen be concentrated at that portion relative to the other portions and the apparent catalytic efficiency be lowered.

The catalyst layer in the inventive methanol production process is not particularly limited as long as it allows hydrogen and carbon dioxide to be contacted with each other and also with the catalyst. For example, the catalyst layer may be a massive layer or may be formed of a plurality of particles or powder. The catalyst layer is preferably formed by packing the copper-containing catalyst with a particle diameter of 3 to 20 mm, namely, particles of the catalyst in the reactor. Further, the methanol production process of the invention may involve a plurality of catalyst layers.

The reactor may be disposed vertically or horizontally in any direction. It is needless to mention that the reactor may be shaped like a vessel or a curved pipe. However, it is preferable that the reactor have a substantially linear tube shape. The upstream and the downstream may be arranged in any positional relationship. For example, the reactor may be a downflow reactor or an upflow reactor.

The inventive production process includes the step (b) in which part or all of the reaction mixture is circulated to the step (a). Preferably, the reaction mixture is returned to the step (a) in such a manner that part or all of methanol and water are separated from the reaction mixture and the residue is returned to the step (a). The residue usually contains unreacted materials (for example, hydrogen, carbon dioxide) and byproducts (for example, carbon monoxide). Further, the residue usually contains inert components, in detail compounds (p) containing carbon and hydrogen except methanol, for example hydrocarbons such as methane and ethane, and may further contain part of methanol and water.

Production of methanol is considered in which a reaction mixture containing methanol is obtained from hydrogen and carbon dioxide and the reaction mixture is subjected to a step in which unreacted materials in the reaction mixture are returned to the reaction step (hereinafter, this returning is referred to as recycle step) (the recycle step corresponding to the step (b) in the inventive production process). In such a process, the reaction mixture will contain little inert components when hydrogen and carbon dioxide used as materials are of high purity. Therefore, it may be considered that performing the recycle step will not cause a decrease in reaction efficiency due to the inert components. In fact, however, it is substantially impossible for the reaction system to be free of highly stable gases such as nitrogen or compounds (p) containing carbon and hydrogen which may be present in the hydrogen and the carbon dioxide as the materials. Such compounds (p) containing carbon and hydrogen which are possibly found in the hydrogen and the carbon dioxide as the materials are, for example, hydrocarbons such as methane, ethane and propane, and most probably methane. This methane often originates from hydrogen as the material. The reason for this is probably because steam reforming of methane is a typical industrial process of hydrogen production. Such compounds do not react with the copper-containing catalyst used in the step (a), and therefore tend to increase their concentrations when the recycle step is repeated. An increase in their concentrations is considered to lead to a decrease in the concentrations of hydrogen and carbon dioxide as the reaction materials as well as to a decrease in methanol productivity. It is needless to mention that the types of compounds (p) containing carbon and hydrogen which may be found in the reaction system can vary in accordance with the origins of the hydrogen and the carbon dioxide as the materials.

In such a case, a possible remedy is to purge part or all of the gas to be returned to the reaction step so as to increase the concentration of hydrogen and carbon dioxide. However, this purge gas should not be released into the atmosphere because the hydrocarbons such as methane are known to have a strong greenhouse effect.

Another possible approach to avoid the above problem is to increase the purities of hydrogen and carbon dioxide beforehand. However, this approach entails a separate facility and the facility has to be large-scaled as the target purities of hydrogen and carbon dioxide are higher, thus leading to an increase in fixed costs.

The process for producing methanol according to the present invention includes a step (c1) of combusting part of the reaction mixture to produce a combustion product and energy, and a step (c2) of circulating part or all of the combustion product to the step (a). These steps are performed when the concentration of the inert components has increased, namely, when the reaction mixture formed in the step (a) comes to contain compounds (p) containing carbon and hydrogen except methanol at not less than 0.1 mol% (wherein all the components of the reaction mixture excluding methanol and water represent 100 mol%).

The step (c1) may be performed such that part of the reaction mixture which contains compounds (p) containing carbon and hydrogen except methanol at not less than 0.1 mol% is combusted directly, or may be performed such that part or all of such components as methanol and water are separated from the reaction mixture and the residue is combusted. Alternatively, the step may be performed such that components such as hydrogen and carbon oxides are separated from the reaction mixture and the residue is combusted.

The reaction mixture usually contains not only the components (p) but also hydrogen and carbon oxides. Performing the step (c1) such that hydrogen is combusted can result in a decrease in the reaction efficiency in methanol production or can cause a possibility for the formed water to be circulated to the step (a). The circulation of water to the step (a) is preferably avoided as much as possible in view of the durability of the catalyst.

If possible, the carbon oxides are preferably prevented from undergoing the step (c1) because the carbon oxides can cause a decrease in combustion efficiency.

For example, hydrogen may be removed before the step (c1) by a so-called cryogenic separation method utilizing the fact that hydrogen is a light-boiling fraction, or by an adsorption desorption method in which part or all of the reaction mixture is subjected to pressure swing separation or in which hydrogen is separated by contact with, for example, a hydrogen storage alloy. For example, the carbon oxides may be removed by being absorbed by water or a basic aqueous solution.

In the step (c2), the combustion product is circulated to the step (a). Preferably, the combustion product that is circulated includes carbon oxides such as carbon monoxide and carbon dioxide.

The combustion product formed in the step (c1) may be directly returned to the step (a). Alternatively, the combustion product may be circulated in such a manner that the carbon oxides in the combustion product are absorbed by, for example, alkaline water to separate typical inert components such as nitrogen and the alkaline water which has absorbed the carbon oxides is treated by heating or the like to recover the carbon oxides, which are then returned to the step (a). In the case where the reaction mixture obtained in the step (a) has a high nitrogen content, it is useful to separate nitrogen by the above method. If nitrogen is separated, the nitrogen gas may be released into the atmosphere. Nitrogen does not allegedly contribute to a greenhouse effect and is considered to cause little loads to the environment.

The steps (c1) and (c2) are carried out when the reaction mixture contains compounds (p) containing carbon and hydrogen except methanol at not less than 0.1 mol%, preferably not less than 0.2 mol% (wherein all the components of the reaction mixture excluding methanol and water represent 100 mol%). If the concentration of the compounds (p) is less than the above range, a decrease in reaction efficiency is small even when the compounds (p) are returned as such to the step (a). If the steps (c1) and (c2) are performed while the concentration of the compounds (p) is below the above range, the amount of energy recovered is small relative to the amount of energy required to perform the steps (c1) and (c2), namely, the efficiency in energy recovery is low.

The steps (c1) and (c2) are performed at any upper-limit concentration of the compounds (p) without limitation. The reaction mixture usually contains compounds (p) containing carbon and hydrogen except methanol at not more than 50 mol%, preferably not more than 40 mol%, and more preferably not more than 30 mol% (wherein all the components of the reaction mixture excluding methanol and water represent 100 mol%). Any concentration of the compounds (p) exceeding the above range indicates that the reaction efficiency in the step (a) has been so markedly lowered that the decrease in reaction efficiency may not be fully complemented even by recovering energy and circulating the combustion product by performing the steps (c1) and (c2).

The process for producing methanol according to the present invention usually includes a step of measuring the content of the compounds (p) in the reaction mixture. To perform the measurement, the reaction mixture obtained in the step (a) may be analyzed directly. When the reaction mixture has been subjected to separation, the analysis may be performed at a plurality of points to determine the content of the compounds (p) in the reaction mixture. For example, the content of the compounds (p) in the reaction mixture may be obtained based on the results of analysis at three points "d", "e" and "f" in EXAMPLES described later.

In the invention, the part of the reaction mixture subjected to the step (c1) preferably represents not less than 0.5 mol%, more preferably not less than 0.8 mol%, and still more preferably not less than 1 mol% of the reaction mixture (wherein all the components of the reaction mixture excluding methanol and water represent 100 mol%). On the other hand, the proportion of the reaction mixture subjected to the step (c1) is preferably not more than 20 mol%, more preferably not more than 15 mol%, and particularly preferably not more than 12 mol%.

If the proportion is excessively smaller than the lower limit, the energy recovery efficiency may be lowered. If the proportion is excessively high, the amount of the combustion of hydrogen in the reaction mixture is so increased that the reaction efficiency can be lowered as a result.

In the present invention, it is not always necessary to perform the steps (c1) and (c2), and 100 mol% of the reaction mixture may be subjected to the step (b) (wherein all the components of the reaction mixture excluding methanol and water represent 100 mol%).

In the case where the content of components (typically nitrogen) other than hydrogen, hydrocarbons and components (p) in the reaction mixture exceeds a specific value, preferably 20 mol%, it is sometimes preferable to purge part or all of such components into the atmosphere.

According to the methanol production process of the invention, methanol can be produced stably using usual hydrogen and carbon dioxide as materials, namely, without the need of the use of highly purified hydrogen and carbon dioxide obtained through high-standard purification steps. That is, methanol is produced while the inert components are removed from the materials by performing the steps (c1) and (c2) in accordance with the concentration of the compounds (p). As a result, methanol can be produced as stably as or more stably than when methanol is produced using high-purity hydrogen and high-purity carbon dioxide. Further, according to the step (c1) in the invention, energy can be obtained by combusting part of the reaction mixture and the resultant combustion product can be used as a material for the production of methanol.

It has often been the case that the compounds (p) described above are released as exhaust gases into the atmosphere or are disposed of after a simple detoxification step such as incineration. The former case and the latter case lead to releasing of greenhouse gases into the atmosphere. In contrast, the methanol production process of the invention utilizes such components, for example the compounds (p), as an energy source for obtaining energy such as combustion heat, and further utilizes carbon oxides that are the combustion product from the combustion of the compounds (p) as materials for methanol production. As already mentioned, methane, which is a typical example of the compounds (p), is known as a stronger greenhouse gas than carbon dioxide. Thus, the present invention unexpectedly achieves not only the suppression of the greenhouse effects of carbon dioxide which has been one of the main objectives, but also the suppression of the greenhouse effects of other greenhouse gases. It can be said that this advantageous effect is achieved because the reaction of interest in the invention is the reaction of carbon oxide and hydrogen into methanol.

In the methanol production process of the invention, part of the reaction mixture is combusted in the step (c1) to produce a combustion product and energy. In this step, the energy may be recovered by a method in which the energy is obtained in the form of thermal energy with a combination of a reactor such as a conventional combustion furnace and a heat exchanger, a method in which the above-obtained thermal energy is converted into electric energy similarly to thermal power generation, or a method in which the energy is recovered as kinetic energy with an internal combustion engine or a similar engine. Of these methods, it is preferable to adopt a method capable of recovering the energy as thermal energy. When the energy is recovered as kinetic energy, it is preferable that the compounds (p) be hydrocarbons having 2 or more carbon atoms such as ethane, propane, butane, hexane and octane.

According to the methanol production process of the invention, methanol can be produced stably with a high production efficiency while suppressing the influences of the purities of the materials.

In the methanol production process of the invention, the step (a) is usually performed at a reaction temperature of 150 to 300°C, a reaction pressure of 1 to 10 MPa-G, and a GHSV (gas hourly space velocity) of 1000 to 30000 hr⁻¹.

For example, the step (a) in the methanol production process may be preferably performed by a so-called upflow method or downflow method using the catalyst layer. Such a method may be carried out using a reactor having the catalyst layer as mentioned above. For example, the reactor may have spaces both upstream and downstream from the catalyst layer, or may be configured such that the upstream side of the catalyst layer is directly connected to a material supply line and the downstream side of the catalyst layer is directly connected to the exit of the reactor (a reaction product collection line).

In the methanol production process of the invention, it is preferable that hydrogen and carbon dioxide be reacted with each other in a reactor having the catalyst layer. The reaction mixture obtained from the exit of the reactor usually contains unreacted hydrogen and carbon dioxide and byproduct carbon monoxide in addition to methanol and water produced by the reaction. The reaction mixture is usually obtained as a gas. That is, the reaction mixture is preferably a gaseous reaction mixture.

The methanol production process of the invention includes a step (d) of separating a component including methanol from the reaction mixture. The step (d) is usually carried out by separating a component including methanol from the gaseous reaction mixture.

The gaseous reaction mixture is usually cooled and thereafter separated into a liquid mixture and a gaseous mixture with a gas liquid separator. This separation is usually performed at a pressure of 0 to 10 MPa-G and a temperature of -10 to 50°C. The separation with a gas liquid separator may be performed multiple times, and may be carried out plural times under different conditions. The liquid mixture obtained by the separation corresponds to the component including methanol and is formed of methanol, water and carbon dioxide dissolved therein. The gaseous mixture includes the unreacted materials, byproduct carbon monoxide and the compounds (p). At least part of the gaseous mixture is circulated back to the reactor through the step (b) and the steps (c1) and (c2).

As mentioned above, the gaseous mixture may contain byproduct carbon monoxide. Carbon monoxide may be supplied to the reactor as an impurity in the materials.

The methanol production process of the invention is characterized by reusing the inert components. This characteristic provides significant effects when the process is performed on a large scale because the greenhouse effects are limited even if gaseous inert components are released into the atmosphere on a small scale as well as because the amount of energy produced by small-scale combustion is small and the energy recovery efficiency will be low.

Thus, the inventive methanol production process is substantially performed on such a large scale that the energy by combustion can be efficiently recovered. The use of a multitubular reactor is preferable from the viewpoint of temperature control such as heat removal. In the case where such a multitubular reactor is used in combination with a fixed-bed catalyst layer, the scale of the catalyst layer per tube is preferably not less than 1 L, more preferably not less than 5 L, and still more preferably not less than 10 L. Specifying the upper limit does not have any essential significance. However, it is usually preferable that the scale be not more than 30 L. Regarding the scale of the catalyst layers combined, the upper limit is preferably 500 m³, more preferably 400 m³, still more preferably 200 m³, and particularly preferably 150 m³, and the lower limit is preferably 1 m³, more preferably 10 m³, and still more preferably 20 m³. A scale in this range ensures a high energy recovery efficiency and is preferable also from the viewpoint of reaction control.

The catalyst layer is frequently used in combination with a temperature control tank shaped like, for example, a jacket. A single catalyst layer may be combined with such a temperature control tank. Alternatively, a plurality of catalyst layers may be stored in one temperature control tank; namely, a multitubular system may be adopted. Such a multitubular system is preferably used for industrial implementation.

As a matter of course, the scale of the reactor is not less than that of the catalyst layers.

In the methanol production process of the invention, the reaction may be terminated by a known method without limitation. In detail, the reaction may be preferably terminated in such a manner that without purging the residual gas in the reaction system with an inert gas or the like, the supply of the material mixture gas is switched to the supply of hydrogen so as to allow hydrogen to react with carbon monoxide and carbon dioxide remaining in the reaction system under similar conditions to those described above, thereby converting substantially the entirety of such residual materials into methanol.

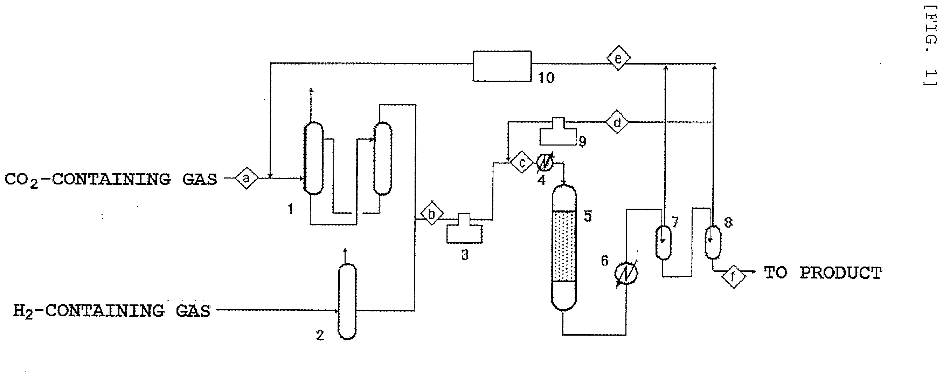

An embodiment of the methanol production process according to the invention will be described below with reference to FIG. 1. Hydrogen as a material gas is passed through a purification device 2 where mainly water and fine powdery solids are to be removed. On the other hand, carbon dioxide is passed through a purification device 1 where mainly water, fine powdery solids and impurities affecting the reaction of interest in the invention are to be removed. Thereafter, these material gases are mixed with each other with a predetermined molar ratio (hydrogen/carbon dioxide). The mixture is pressurized to an appropriate pressure with a booster compressor 3, heated to an appropriate temperature with a heater 4, and fed to a reaction circulation system. The material mixture gas that has entered the reaction circulation system is led to a reactor 5 and is subjected to a methanol synthesis reaction. The reactor includes a catalyst layer formed of the copper-containing catalyst. In the reactor 5, hydrogen and carbon dioxide are reacted with each other to form a gaseous reaction mixture containing methanol. The gaseous reaction mixture (the reaction mixture gas) discharged from the reactor 5 is cooled to normal temperature or below with a condenser 6, where mainly the formed methanol and water are liquefied. The mixture is then led to gas liquid separators 7 and 8. After the mixture is separated into a liquid mixture containing methanol and water, and a gaseous mixture in the gas liquid separators 7 and 8, these mixtures are each withdrawn and water and methanol are further separated from each other. A major proportion of the gaseous mixture is circulated to the reactor 5 with a circulation compressor 9. When the concentration of compounds (p) has exceeded a predetermined concentration, at least part of the gaseous mixture is led to an apparatus 10 equipped with a combustion device and a heat recovery device. In the apparatus 10, the gaseous mixture is combusted and the heat is recovered. The combustion product is then circulated to the reactor 5. In the case where the gas supplied from the apparatus 10 contains components such as nitrogen which do not participate in the combustion reaction, such components may be separated and released into the atmosphere.

Although not illustrated in FIG. 1, the liquid mixture withdrawn from the gas liquid separators 7 and 8 may be appropriately treated so as to separate components such as carbon dioxide dissolved in the liquid mixture. In such a case, the separated gas may be discharged from the system or may be returned to the reaction circulation system.

### EXAMPLES

The present invention will be described in greater detail by presenting examples hereinbelow without limiting the scope of the invention.

A system illustrated in FIG. 1 was used in EXAMPLES. The system included a carbon dioxide purification device 1 for purifying carbon dioxide as a material, a hydrogen purification device 2 for purifying hydrogen as a material, a booster compressor 3 for supplying a mixture gas, a heater 4 for heating the mixture gas, a reactor 5 for performing the reaction, a condenser 6 for cooling a reaction mixture, gas liquid separators 7 and 8 for separating the reaction mixture into a liquid and a gas, a circulation compressor 9 for circulating at least part of the separated gas to the reactor 5, and an apparatus 10 for combusting at least part of the separated gas and recovering the combustion heat.

### [PRODUCTION EXAMPLE 1]

### (Preparation of copper-containing catalyst)

A catalyst was prepared substantially in accordance with the method described in EXAMPLE 1 of Patent Literature 3 (JP-A-H10-309466).

The composition of the catalyst was CuO: 45.2 wt%, ZnO: 27.1 wt%, Al₂O₃: 4.5 wt%, ZrO₂: 22.6 wt% and SiO₂: 0.6 wt%.

### [EXAMPLE 1]

With the use of the system illustrated in FIG. 1, the reaction was performed for 24 hours after a steady state was reached under the following conditions.

Reaction pressure: 5.0 MPa-G
Reaction temperature: 250°C
GHSV: 10000 hr⁻¹
Catalyst packing scale: 16.6 L

The composition, the temperature and the pressure were measured at analytical points ("a" to "f") in FIG. 1 with a process gas chromatograph, a thermometer and a pressure meter.

During the operation, methane which was probably present in the material hydrogen and corresponded to a compound (p) defined in the present invention accumulated, and a 8 mol% portion of a gas (a gaseous mixture) separated in the gas liquid separator 7 and the gas liquid separator 8 was introduced into the apparatus 10 in FIG. 1. (Hereinafter, this introduction will be sometimes referred to as recycle gas purging. The recycle gas purging rate in this case is 8 mol%.) The introduced gas was then combusted and the heat was recovered. The resultant gas containing carbon oxides was returned to the reaction step. The steady state was considered to have been reached when the methane concentration at the point "b" became 5.7 mol% and the methane concentration at the point "d" became 17 mol% (wherein all the components of the gas excluding methanol and water represented 100 mol%) during the above operation. The measurement results are described in Table 1.
[Table 1]

**Table 1**

| | a | b | c | d | e | f |
|---|---|---|---|---|---|---|
| H2/(kmol/hr) | 0.00 | 1.14 | 5.12 | 3.98 | 0.35 | 0.00 |
| CO2/(kmol/hr) | 0.30 | 0.34 | 1.07 | 0.73 | 0.07 | 0.00 |
| CO/(kmol/hr) | 0.00 | 0.00 | 0.13 | 0.13 | 0.01 | 0.00 |
| Methanol/(kmol/hr) | 0.00 | 0.00 | 0.02 | 0.02 | 0.00 | 0.26 |
| Water/(kmol/hr) | 0.20 | 0.02 | 0.03 | 0.01 | 0.00 | 0.29 |
| Methane/(kmol/hr) | 0.00 | 0.09 | 1.08 | 0.99 | 0.09 | 0.00 |
| N2/(kmol/hr) | 4.43 | 0.00 | 0.00 | 0.00 | 0.00 | 0.00 |
| Total/(kmol/hr) | 4.93 | 1.59 | 7.45 | 5.86 | 0.52 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

In the above methanol production process, the methanol conversion based on CO₂ used was 88 mol%, and the methanol output per unit volume of the catalyst was 16 mol-methanol/L-Cat/hr. The amount of heat recovered at the combustion heat recovery device was 480 MJ/mol-methanol.

### [EXAMPLE 2]

The reaction was performed for 24 hours in the same manner as in EXAMPLE 1 after a steady state was reached under the following conditions.

Reaction pressure: 5.0 MPa-G
Reaction temperature: 250°C
GHSV: 10000 hr⁻¹
Catalyst packing scale: 14.5 L
Methane concentration at point "b": 1.3 mol%

Methane concentration at point "d": 3.4 mol%
Recycle gas purging rate: 8 mol%

The results of measurements at the points "a" to "f" are described in Table 2.
[Table 2]

**Table 2**

| | a | b | c | d | e | f |
|---|---|---|---|---|---|---|
| H2/(kmol/hr) | 0.00 | 1.14 | 5.12 | 3.98 | 0.35 | 0.00 |
| CO2/(kmol/hr) | 0.30 | 0.34 | 1.07 | 0.73 | 0.07 | 0.00 |
| CO/(kmol/hr) | 0.00 | 0.00 | 0.13 | 0.13 | 0.01 | 0.00 |
| Methanol/(kmol/hr) | 0.00 | 0.00 | 0.02 | 0.02 | 0.00 | 0.26 |
| Water/(kmol/hr) | 0.20 | 0.02 | 0.03 | 0.01 | 0.00 | 0.29 |
| Methane/(kmol/hr) | 0.00 | 0.02 | 0.19 | 0.17 | 0.02 | 0.00 |
| N2/(kmol/hr) | 4.43 | 0.00 | 0.00 | 0.00 | 0.00 | 0.00 |
| Total/(kmol/hr) | 4.93 | 1.52 | 6.56 | 5.04 | 0.45 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

The methanol conversion based on CO₂ used was 88 mol%, and the methanol output per unit volume of the catalyst was 18 mol-methanol/L-Cat/hr. The amount of heat recovered at the combustion heat recovery device was 310 MJ/mol-methanol.

### [EXAMPLE 3]

The reaction was performed for 24 hours in the same manner as in EXAMPLE 1 after a steady state was reached under the following conditions.

Reaction pressure: 5.0 MPa-G
Reaction temperature: 250°C
GHSV: 10000 hr⁻¹
Catalyst packing scale: 14.5 L
Methane concentration at point "b": 0.1 mol%
Methane concentration at point "d": 0.3 mol%
Recycle gas purging rate: 8 mol%

The results of measurements at the analytical points ("a" to "f") in FIG. 1 are described in Table 3.
[Table 3]

**Table 3**

| | a | b | c | d | e | f |
|---|---|---|---|---|---|---|
| H2/(kmol/hr) | 0.00 | 1.14 | 5.12 | 3.98 | 0.35 | 0.00 |
| CO2/(kmol/hr) | 0.30 | 0.34 | 1.07 | 0.73 | 0.07 | 0.00 |
| CO/(kmol/hr) | 0.00 | 0.00 | 0.13 | 0.13 | 0.01 | 0.00 |
| Methanol/(kmol/hr) | 0.00 | 0.00 | 0.02 | 0.02 | 0.00 | 0.26 |
| Water/(kmol/hr) | 0.20 | 0.02 | 0.03 | 0.01 | 0.00 | 0.29 |
| Methane/(kmol/hr) | 0.00 | 0.001 | 0.014 | 0.013 | 0.001 | 0.00 |
| N2/(kmol/hr) | 4.43 | 0.00 | 0.00 | 0.00 | 0.00 | 0.00 |
| Total/(kmol/hr) | 4.93 | 1.501 | 6.384 | 4.883 | 0.43 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

The methanol conversion based on CO₂ used was 88 mol%, and the methanol output per unit volume of the catalyst was 18 mol-methanol/L-Cat/hr. The amount of heat recovered at the combustion heat recovery device was 280 MJ/mol-methanol.

### [COMPARATIVE EXAMPLES]

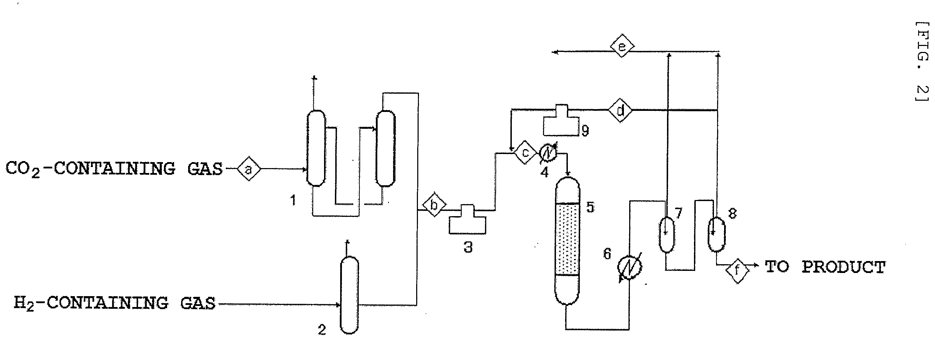

A system illustrated in FIG. 2 was used in COMPARATIVE EXAMPLES. The system included a carbon dioxide purification device 1 for purifying carbon dioxide as a material, a hydrogen purification device 2 for purifying hydrogen as a material, a booster compressor 3 for supplying a mixture gas, a heater 4 for heating the mixture gas, a reactor 5 for performing the reaction, a condenser 6 for cooling a reaction mixture, gas liquid separators 7 and 8 for separating the reaction mixture into a liquid and a gas, and a circulation compressor 9 for circulating at least part of the separated gas to the reactor 5. Part of the gas in the system was discharged downstream from the analytical point "e" to the outside without combusting the gas or recovering the heat. (This corresponded to recycle gas purging.)

### [COMPARATIVE EXAMPLE 1]

With the use of the system illustrated in FIG. 2, the reaction was performed for 24 hours in the same manner as in EXAMPLE 1 after a steady state was reached under the following conditions, except that part of the gas was discharged (recycle gas purged) from the system.

Reaction pressure: 5.0 MPa-G
Reaction temperature: 250°C
GHSV: 10000 hr⁻¹
Catalyst packing scale: 16.6 L
Methane concentration at point "b": 5.6 mol%
Methane concentration at point "d": 17 mol%
Recycle gas purging rate: 8 mol%

The results of measurements at the analytical points ("a" to "f") in FIG. 2 are described in Table 4.
[Table 4]

**Table 4**

| | a | b | c | d | e | f |
|---|---|---|---|---|---|---|
| H2/(kmol/hr) | 0.00 | 1.14 | 5.12 | 3.98 | 0.35 | 0.00 |
| CO2/(kmol/hr) | 0.36 | 0.34 | 1.07 | 0.73 | 0.07 | 0.00 |
| CO/(kmol/hr) | 0.00 | 0.00 | 0.13 | 0.13 | 0.01 | 0.00 |
| Methanol/(kmol/hr) | 0.00 | 0.00 | 0.02 | 0.02 | 0.00 | 0.26 |
| Water/(kmol/hr) | 0.24 | 0.02 | 0.03 | 0.01 | 0.00 | 0.29 |
| Methane/(kmol/hr) | 0.00 | 0.09 | 1.08 | 0.99 | 0.09 | 0.00 |
| N2/(kmol/hr) | 5.43 | 0.00 | 0.00 | 0.00 | 0.00 | 0.00 |
| Total/(kmol/hr) | 6.03 | 1.59 | 7.45 | 5.86 | 0.52 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

The methanol conversion based on CO₂ used was 72 mol%, and the methanol output per unit volume of the catalyst was 16 mol-methanol/L-Cat/hr.

The methanol conversion was lower than those in EXAMPLES. As a matter of course, the amount of recovered heat was zero. These results are probably because hydrogen and carbon dioxide as well as methane were discharged to the outside of the system and consequently there occurred no conversion of methane-derived carbon dioxide into methanol.

### [COMPARATIVE EXAMPLE 2]

With the use of the system illustrated in FIG. 2, the reaction was performed for 24 hours in the same manner as in COMPARATIVE EXAMPLE 1 after a steady state was reached under the following conditions.

Reaction pressure: 5.0 MPa-G
Reaction temperature: 250°C
GHSV: 10000 hr⁻¹
Catalyst packing scale: 28.5 L
Methane concentration at point "b": 4.9 mol%
Methane concentration at point "d": 49 mol%
Recycle gas purging rate: 1 mol%

The results of measurements at the analytical points ("a" to "f") in FIG. 2 under the steady state are described in Table 5.
[Table 5]

**Table 5**

| | a | b | c | d | e | f |
|---|---|---|---|---|---|---|
| H2/(kmol/hr) | 0.00 | 0.85 | 5.12 | 4.27 | 0.06 | 0.00 |
| CO2/(kmol/hr) | 0.30 | 0.29 | 1.07 | 0.78 | 0.01 | 0.00 |
| CO/(kmol/hr) | 0.00 | 0.00 | 0.14 | 0.14 | 0.00 | 0.00 |
| Methanol/(kmol/hr) | 0.00 | 0.00 | 0.02 | 0.02 | 0.00 | 0.26 |
| Water/(kmol/hr) | 0.20 | 0.02 | 0.03 | 0.01 | 0.00 | 0.29 |
| Methane/(kmol/hr) | 0.00 | 0.06 | 5.00 | 4.94 | 0.06 | 0.00 |
| N2/(kmol/hr) | 4.58 | 0.00 | 0.00 | 0.00 | 0.00 | 0.00 |
| Total/(kmol/hr) | 5.08 | 1.22 | 11.38 | 10.16 | 0.13 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

The methanol conversion based on CO₂ used was 86 mol%, and the methanol output per unit volume of the catalyst was 9 mol-methanol/L-Cat/hr.

### [COMPARATIVE EXAMPLE 3]

With the use of the system illustrated in FIG. 2, the reaction was performed for 24 hours in the same manner as in COMPARATIVE EXAMPLE 1 after a steady state was reached under the following conditions.

Reaction pressure: 5.0 MPa-G
Reaction temperature: 250°C
GHSV: 10000 hr⁻¹
Catalyst packing scale: 14.5 L
Methane concentration at point "b": 1.3 mol%
Methane concentration at point "d": 3.4 mol%
Recycle gas purging rate: 8 mol%

The results of measurements at the analytical points ("a" to "f") in FIG. 2 under the steady state are described in Table 6.
[Table 6]

**Table 6**

| | a | b | c | d | e | f |
|---|---|---|---|---|---|---|
| H2/(kmol/hr) | 0.00 | 1.14 | 5.12 | 3.98 | 0.35 | 0.00 |
| CO2/(kmol/hr) | 0.36 | 0.34 | 1.07 | 0.73 | 0.07 | 0.00 |
| CO/(kmol/hr) | 0.00 | 0.00 | 0.13 | 0.13 | 0.01 | 0.00 |
| Methanol/(kmol/hr) | 0.00 | 0.00 | 0.02 | 0.02 | 0.00 | 0.26 |
| Water/(kmol/hr) | 0.24 | 0.02 | 0.03 | 0.01 | 0.00 | 0.29 |
| Methane/(kmol/hr) | 0.00 | 0.02 | 0.19 | 0.17 | 0.02 | 0.00 |
| N2/(kmol/hr) | 5.43 | 0.00 | 0.00 | 0.00 | 0.00 | 0.00 |
| Total/(kmol/hr) | 6.03 | 1.52 | 6.56 | 5.04 | 0.45 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

The methanol conversion based on CO₂ used was 72 mol%, and the methanol output per unit volume of the catalyst was 18 mol-methanol/L-Cat/hr.

### [REFERENCE EXAMPLE 1]

With the use of the system illustrated in FIG. 2, the reaction was performed for 24 hours in the same manner as in COMPARATIVE EXAMPLE 1 after a steady state was reached under the following conditions.

Reaction pressure: 5.0 MPa-G
Reaction temperature: 250°C
GHSV: 10000 hr⁻¹
Catalyst packing scale: 16.6 L
Methane concentration at point "b": 1 mol%
Methane concentration at point "d": 18 mol%
Recycle gas purging rate: 1 mol%

The composition, the temperature and the pressure were measured at the analytical points ("a" to "f") in FIG. 2 under the steady state. The results are described in Table 7.
[Table 7]

**Table 7**

| | a | b | c | d | e | f |
|---|---|---|---|---|---|---|
| H2/(kmol/hr) | 0.00 | 0.84 | 5.12 | 4.28 | 0.04 | 0.00 |
| CO2/(kmol/hr) | 0.30 | 0.29 | 1.07 | 0.78 | 0.01 | 0.00 |
| CO/(kmol/hr) | 0.00 | 0.00 | 0.14 | 0.14 | 0.00 | 0.00 |
| Methanol/(kmol/hr) | 0.00 | 0.00 | 0. 02 | 0.02 | 0.00 | 0.26 |
| Water/(kmol/hr) | 0.20 | 0.02 | 0.03 | 0.01 | 0.00 | 0.29 |
| Methane/(kmol/hr) | 0.00 | 0.01 | 1.12 | 1.11 | 0.01 | 0.00 |
| N2/(kmol/hr) | 4.56 | 0.00 | 0.00 | 0.00 | 0.00 | 0.00 |
| Total/(kmol/hr) | 5.06 | 1.16 | 7.50 | 6.34 | 0.06 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

The methanol conversion from CO₂ was 86 mol%, and the methanol output per unit volume of the catalyst was 16 mol-methanol/L-Cat/hr.

### [COMPARATIVE EXAMPLE 4]

With the use of the system illustrated in FIG. 2, the reaction was performed for 24 hours in the same manner as in COMPARATIVE EXAMPLE 1 after a steady state was reached under the following conditions.

Reaction pressure: 5.0 MPa-G
Reaction temperature: 250°C
GHSV: 10000 hr⁻¹
Catalyst packing scale: 14.5 L
Methane concentration at point "b": 0.1 mol%
Methane concentration at point "d": 0.3 mol%
Recycle gas purging rate: 8 mol%

The results of measurements at the analytical points ("a" to "f") in FIG. 2 under the steady state are described in Table 8.
[Table 8]

**Table 8**

| | a | b | c | d | e | f |
|---|---|---|---|---|---|---|
| H2/(kmol/hr) | 0.00 | 1.14 | 5.12 | 3. 98 | 0.35 | 0.00 |
| CO2/(kmol/hr) | 0.36 | 0.34 | 1.07 | 0.73 | 0.07 | 0.00 |
| CO/(kmol/hr) | 0.00 | 0.00 | 0.13 | 0. 13 | 0.01 | 0.00 |
| Methanol/(kmol/hr) | 0.00 | 0.00 | 0.02 | 0.02 | 0.00 | 0.26 |
| Water/(kmol/hr) | 0.24 | 0.02 | 0.03 | 0.01 | 0.00 | 0.29 |
| Methane/(kmol/hr) | 0.00 | 0.001 | 0.014 | 0.013 | 0.001 | 0.00 |
| N2/(kmol/hr) | 5.43 | 0.00 | 0.00 | 0.00 | 0.00 | 0.00 |
| Total/(kmol/hr) | 6.03 | 1.501 | 6.384 | 4.883 | 0.431 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

The methanol conversion from CO₂ was 72 mol%, and the methanol output per unit volume of the catalyst was 18 mol-methanol/L-Cat/hr.

### [REFERENCE EXAMPLE 2]

With the use of the system illustrated in FIG. 2, the reaction was performed for 24 hours in the same manner as in COMPARATIVE EXAMPLE 1 after a steady state was reached under the following conditions.

Reaction pressure: 5.0 MPa-G
Reaction temperature: 250°C
GHSV: 10000 hr⁻¹
Catalyst packing scale: 14.5 L
Methane concentration at point "b": 0.1 mol%
Methane concentration at point "d": 1.6 mol%
Recycle gas purging rate: 8 mol%

The results of measurements at the analytical points ("a" to "f") in FIG. 2 under the steady state are described in Table 9.
[Table 9]

**Table 9**

| | a | b | c | d | e | f |
|---|---|---|---|---|---|---|
| H2/(kmol/hr) | 0.00 | 0.84 | 5.12 | 4.28 | 0.04 | 0.00 |
| CO2/(kmol/hr) | 0.30 | 0.29 | 1.07 | 0.78 | 0.01 | 0.00 |
| CO/(kmol/hr) | 0.00 | 0.00 | 0.14 | 0.14 | 0.00 | 0.00 |
| Methanol/(kmol/hr) | 0.00 | 0.00 | 0.02 | 0.02 | 0.00 | 0.26 |
| Water/(kmol/hr) | 0.20 | 0.02 | 0.03 | 0.01 | 0.00 | 0.29 |
| Methane/(kmol/hr) | 0.00 | 0.001 | 0.084 | 0.083 | 0.001 | 0.00 |
| N2/(kmol/hr) | 4.56 | 0.00 | 0.00 | 0.00 | 0.00 | 0.00 |
| Total/(kmol/hr) | 5.06 | 1.151 | 6.464 | 5.313 | 0.051 | 0.55 |
| Temperature/°C | 25 | 25 | 25 | 25 | 25 | 25 |
| Pressure/MPa-G | 0.00 | 0.76 | 5.00 | 4.60 | 0.10 | 0.00 |

The methanol conversion from CO₂ was 86 mol%, and the methanol output per unit volume of the catalyst was 18 mol-methanol/L-Cat/hr.

The results in COMPARATIVE EXAMPLES and REFERENCE EXAMPLES, as well as the results in EXAMPLES show that even when the proportion of inert components is increased during the production of methanol, the inventive process can realize a methanol yield and a carbon dioxide conversion comparable to when little inert components are present.

## Claims

1. A process for producing methanol, comprising:
a step (a) of reacting hydrogen and carbon dioxide with each other in a reactor in the presence of a copper-containing catalyst to produce a reaction mixture containing methanol;
a step (b) of circulating part or all of the reaction mixture to the step (a); and then
a step (c1) of combusting part of the reaction mixture to produce a combustion product and energy, and a step (c2) of circulating part or all of the combustion product to the step (a), the step (c1) and the step (c2) being performed when the reaction mixture contains a compound (p) containing carbon and hydrogen except methanol at not less than 0.1 mol% (wherein all components of the reaction mixture excluding methanol and water represent 100 mol%); and
a step (d) of separating a component including methanol from the reaction mixture.

2. The process for producing methanol according to Claim 1, wherein the compound (p) is inert to the copper-containing catalyst.

3. The process for producing methanol according to Claim 1 or 2, wherein at least part of the compounds (p) is a hydrocarbon.

4. The process for producing methanol according to Claim 1 or 2, wherein at least part of the compounds (p) is methane.

5. The process for producing methanol according to Claim 1 or 2, wherein the compound (p) is methane.

6. The process for producing methanol according to any one of Claims 1 to 5, wherein the reaction mixture contains the compound (p) at 0.1 to 50 mol%.

7. The process for producing methanol according to any one of Claims 1 to 6, wherein the copper-containing catalyst is a catalyst containing copper, zinc, aluminum and silicon.

8. The process for producing methanol according to any one of Claims 1 to 7, wherein the energy produced in the step (c1) is recovered in the form of energy selected from thermal energy, electric energy and kinetic energy.

9. The process for producing methanol according to any one of Claims 1 to 7, wherein the energy produced in the step (c1) is recovered as thermal energy.

## Patentansprüche

1. Verfahren zur Herstellung von Methanol, umfassend:
einen Schritt (a) des Reagierens von Wasserstoff und Kohlendioxid miteinander in einem Reaktor in Anwesenheit von einem kupferhaltigen Katalysator, um ein Reaktionsgemisch herzustellen, das Methanol enthält;
einen Schritt (b) des Zirkulierens von einem Teil oder dem gesamten Reaktionsgemisch zu Schritt (a); und dann
einen Schritt (c1) des Verbrennens von einem Teil des Reaktionsgemischs, um ein Verbrennungsprodukt und Energie herzustellen, und einen Schritt (c2) des Zirkulierens von einem Teil oder dem gesamten Reaktionsgemisch zu dem Schritt (a), wobei der Schritt (c1) und der Schritt (c2) durchgeführt werden, wenn das Reaktionsgemisch eine Verbindung (p) enthält, die Kohlenstoff und Wasserstoff mit Ausnahme von Methanol von nicht weniger als 0,1 Mol-% enthält (wobei alle Komponenten des Rektionsgemischs mit Ausnahme von Methanol und Wasser 100 Mol-% darstellen); und
einen Schritt (d) des Trennens von einem Komponenten einschließlich Methanol von dem Reaktionsgemisch.

2. Verfahren zur Herstellung von Methanol nach Anspruch 1, wobei die Verbindung (p) gegenüber dem kupferhaltigen Katalysator inert ist.

3. Verfahren zur Herstellung von Methanol nach Anspruch 1 oder 2, wobei mindestens ein Teil der Verbindungen (p) ein Kohlenwasserstoff ist.

4. Verfahren zur Herstellung von Methanol nach Anspruch 1 oder 2, wobei mindestens ein Teil der Verbindungen (p) Methan ist.

5. Verfahren zur Herstellung von Methanol nach Anspruch 1 oder 2, wobei die Verbindung (p) Methan ist.

6. Verfahren zur Herstellung von Methanol nach einem der Ansprüche 1 bis 5, wobei das Reaktionsgemisch die Verbindung (p) zu 0,1 bis 50 Mol-% enthält.

7. Verfahren zur Herstellung von Methanol nach einem der Ansprüche 1 bis 6, wobei der kupferhaltige Katalysator ein Katalysator ist, der Kupfer, Zink, Aluminium und Silizium enthält.

8. Verfahren zur Herstellung von Methanol nach einem der Ansprüche 1 bis 7, wobei die Energie, die in Schritt (c1) hergestellt wird, in Form von Energie zurückgewonnen wird, die ausgewählt ist aus thermischer Energie, elektrischer Energie und kinetischer Energie.

9. Verfahren zur Herstellung von Methanol nach einem der Ansprüche 1 bis 7, wobei die Energie, die in Schritt (c1) hergestellt wird, als thermische Energie zurückgewonnen wird.

## Revendications

1. Procédé de production de méthanol, comprenant :
une étape (a) de mise en réaction d'hydrogène et de dioxyde de carbone l'un avec l'autre dans un réacteur en présence d'un catalyseur contenant du cuivre pour produire un mélange réactionnel contenant du méthanol ;
une étape (b) de mise en circulation de tout ou partie du mélange réactionnel vers l'étape (a) ; et puis
une étape (c1) de combustion d'une partie du mélange réactionnel pour produire un produit de combustion et de l'énergie, et une étape (c2) de mise en circulation de tout ou partie du produit de combustion vers l'étape (a), l'étape (c1) et l'étape (c2) étant effectuées lorsque le mélange réactionnel contient un composé (p) contenant du carbone et de l'hydrogène à l'exception de méthanol à non moins de 0,1 % en mole (tous les composants du mélange réactionnel à l'exclusion du méthanol et de l'eau représentant 100 % en mole) ; et
une étape (d) de séparation d'un composant comprenant du méthanol du mélange réactionnel.

2. Procédé de production de méthanol selon la revendication 1, dans lequel le composé (p) est inerte pour le catalyseur contenant du cuivre.

3. Procédé de production de méthanol selon la revendication 1 ou 2, dans lequel au moins une partie des composés (p) est un hydrocarbure.

4. Procédé de production de méthanol selon la revendication 1 ou 2, dans lequel au moins une partie des composés (p) est du méthane.

5. Procédé de production de méthanol selon la revendication 1 ou 2, dans lequel le composé (p) est du méthane.

6. Procédé de production de méthanol selon l'une quelconque des revendications 1 à 5, dans lequel le mélange réactionnel contient le composé (p) à raison de 0,1 à 50 % en mole.

7. Procédé de production de méthanol selon l'une quelconque des revendications 1 à 6, dans lequel le catalyseur contenant du cuivre est un catalyseur contenant du cuivre, du zinc, de l'aluminium et du silicium.

8. Procédé de production de méthanol selon l'une quelconque des revendications 1 à 7, dans lequel l'énergie produite dans l'étape (c 1) est récupérée sous forme d'énergie choisie parmi l'énergie thermique, l'énergie électrique et l'énergie cinétique.

9. Procédé de production de méthanol selon l'une quelconque des revendications 1 à 7, dans lequel l'énergie produite dans l'étape (c1) est récupérée sous forme d'énergie thermique.
